(19) Europäisches Patentamt
European Patent Office
Office européen des brevets

(11) EP 1 038 519 B1

(12) **FASCICULE DE BREVET EUROPEEN**

(45) Date de publication et mention de la délivrance du brevet:
**07.06.2006 Bulletin 2006/23**

(51) Int Cl.:
***A61K 8/899*** *(2006.01)* ***A61Q 19/08*** *(2006.01)*

(21) Numéro de dépôt: **00400535.1**

(22) Date de dépôt: **25.02.2000**

(54) **Utilisation comme agent tenseur d'au moins un polymère siliconé greffé**

Verwendung von mindestens einem Propfsilicon als Spannhilfstoff

Use of at least a grafted silicone polymer as a tensioner

(84) Etats contractants désignés:
**AT BE CH CY DE DK ES FI FR GB GR IE IT LI LU MC NL PT SE**

(30) Priorité: **22.03.1999 FR 9903531**

(43) Date de publication de la demande:
**27.09.2000 Bulletin 2000/39**

(73) Titulaire: **L'OREAL**
**75008 Paris (FR)**

(72) Inventeurs:
- **Toumi, Béatrice**
  **92250 La Garenne Colombes (FR)**
- **Mougin, Nathalie**
  **75011 Paris (FR)**
- **Garson, Jean-Claude**
  **92150 Suresnes (FR)**

(74) Mandataire: **Kromer, Christophe**
**L'OREAL - D.I.P.I.**
**25-29 Quai Aulagnier**
**92600 Asnières (FR)**

(56) Documents cités:
**EP-A- 0 582 152**
**WO-A-98/09608**
**WO-A-98/44906**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 038 519 B1

Printed by Jouve, 75001 PARIS (FR)

## Description

La présente invention se rapporte à l'utilisation comme agent tenseur, dans et/ou pour la fabrication d'une composition destinée à la diminution ou à l'effacement des signes du vieillissement, en particulier à la réduction ou à l'effacement des rides et/ou ridules et/ou au lissage de la peau humaine, d'au moins un polymère à squelette polysiloxanique greffé par au moins un monomère organique non-siliconé.

Au cours du processus de vieillissement, il apparaît différents signes sur la peau, très caractéristiques de ce vieillissement, se traduisant notamment par une modification de la structure et des fonctions cutanées. Les principaux signes du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope.

Il est connu de réduire ces signes du vieillissement en utilisant des compositions cosmétiques ou dermatologiques contenant des actifs capables de lutter contre le vieillissement, tels que les $\alpha$-hydroxy-acides, les $\beta$-hydroxy-acides et les rétinoïdes. Ces actifs agissent sur les rides, notamment en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés, conduisant rapidement à un lissage des rides et ridules et à la disparition des marques de fatigue.

Il a donc été proposé, dans l'art antérieur, différentes compositions à effet tenseur permettant d'obtenir un lissage immédiat de la peau.

Ces compositions contiennent généralement, comme agent tenseur, des substances d'origine naturelle, telles que les dérivés végétaux, d'oeufs, de lait ou d'animaux. Ainsi, FR-A-2 758 083 divulgue l'utilisation comme tenseur d'un système polymérique ayant des propriétés physico-chimiques particulières et comprenant au moins un polymère d'origine naturelle, tel qu'un extrait de protéine de soja ou des dérivés de chitine ou de kératine. En outre, la demande de brevet WO 96/19180 décrit des compositions raffermissantes comprenant un agent filmogène contenant au moins un polysaccharide végétal et de la caséine hydrolysée comme actifs tenseurs. Toutefois, l'utilisation de substances d'origine naturelle est limitée par les risques d'encéphalopathie spongiforme bovine. De plus, outre qu'il n'est pas toujours reproductible, l'effet tenseur que procurent ces substances n'est pas très important sur le plan qualitatif et, sur le plan quantitatif, il présente une faible rémanence.

D'autres compositions à effet tenseur de l'art antérieur utilisent des polymères synthétiques. On connaît ainsi de FR-A-2 758 084 une composition à effet tenseur comprenant une dispersion aqueuse d'un système polymérique contenant au moins un polymère d'origine synthétique, choisi parmi différents types de polyuréthannes, les polyurées, les polymères ou copolymères acryliques, les polymères d'acide isophtalique sulfoné et leurs mélanges. Le toucher cosmétique de ces compositions n'est cependant pas toujours satisfaisant.

On connaît également de WO 98/44906 des compositions, notamment à effet tenseur de la peau, comprenant un matériau hybride réticulé filmogène, qui est un polymère comportant une portion inorganique et une portion organique qui peut être constituée d'un polyorganosiloxane portant des greffons acryliques. Ces polymères sont très difficiles à mettre en oeuvre.

Par conséquent, il subsiste toujours le besoin de composés offrant un effet tenseur immédiat, suffisant et durable, sans risque pour le consommateur.

La présente invention a donc pour objet l'utilisation d'un polymère siliconé greffé permettant d'obtenir cet effet.

Plus particulièrement, la présente invention a pour objet l'utilisation, dans une composition cosmétique destinée à diminuer ou effacer les signes de vieillissement cutané, et plus particulièrement à réduire ou effacer les rides et/ou les ridules de la peau et/ou lisser la peau, d'au moins un polymère à squelette polysiloxanique greffé par au moins un monomère organique non-siliconé .

L'invention a également pour objet l'utilisation comme agent tenseur, dans une composition cosmétique, d'au moins un polymère à squelette polysiloxanique greffé par au moins un monomère organique non-siliconé.

Dans le cadre de cette description et des revendications annexées, on entend par « agent tenseur » des composés susceptibles d'avoir un effet tenseur apparent, c'est-à-dire de lisser la peau et réduire, voire faire disparaître, de façon immédiate les rides et les ridules.

L'invention a encore pour objet l'utilisation, pour la fabrication d'une composition destinée à diminuer ou effacer les signes de vieillissement cutané, en particulier à réduire ou effacer les rides et/ou les ridules de la peau et/ou à lisser la peau, d'au moins un polymère à squelette polysiloxanique greffé par au moins un monomère organique non-siliconé.

La composition utilisée dans la présente invention contient, en plus du polymère siliconé greffé précité, un milieu physiologiquement acceptable, c'est-à-dire compatible avec la peau et ses phanères, les muqueuses et les semi-muqueuses.

La quantité de polymère siliconé greffé présente dans la composition est fonction de l'effet tenseur que l'on souhaite conférer à cette composition et elle représente, en général, de 0,03 à 25%, et de préférence de 0,3 à 6%,

mieux, environ 2%, du poids total de la composition.

La présente invention se rapporte également à un procédé de traitement cosmétique d'une peau vieillie, notamment ridée, comprenant l'application sur ladite peau d'au moins un polymère siliconé greffé tel que mentionné précédemment, en une quantité efficace pour estomper la ride par effet tenseur.

Les polymères siliconés greffés mentionnés ci-dessus sont bien connus de l'homme du métier. On connaît ainsi de EP 848 941 l'utilisation de dérivés siliconés greffés anioniques en tant que matériaux fixants, dans un aérosol tel qu'une laque, pour mettre en forme et/ou maintenir la coiffure. Ces composés sont également utilisés pour améliorer l'effet coiffant de compositions capillaires détergentes contenant au moins un polymère cationique, telles que celles décrites dans EP 756 860 et WO 97/46210.

Comme indiqué ci-dessus, les polymères siliconés greffés selon l'invention sont des polymères à squelette polysiloxanique greffé par au moins un monomère organique non-siliconé et leurs mélanges.

Ces polymères siliconés greffés vont maintenant être décrits plus en détail.

A titre préliminaire, les définitions suivantes sont données :

1. "silicone" ou "polysiloxane" : dans le cadre de cette description, on entend par ce terme, en conformité avec l'acception générale, tout polymère ou oligomère organosilicié à structure linéaire ou cyclique, ramifiée ou réticulée, de poids moléculaire variable, obtenu par polymérisation et/ou polycondensation de silanes convenablement fonctionnalisés, et constitué pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyle notamment en $C_1$-$C_{10}$ et en particulier méthyle, les radicaux fluoroalkyle, les radicaux aryle et en particulier phényle, et les radicaux alcényle et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyle ou hydroxyalkyle, les groupements aminés substitués ou non, les groupements amides, les radicaux acyloxy ou acyloxyalkyle, les radicaux hydroxyalkylamino ou aminoalkyle, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

2. "macromère polysiloxane" : en conformité avec l'acception générale, cette expression désigne, dans la présente description, tout monomère contenant dans sa structure une chaîne polymère du type polysiloxane.

Selon la présente invention, le ou les polymères siliconés greffés, à squelette polysiloxanique greffé par des monomères organiques non-siliconés, comprennent une chaîne principale de silicone (ou polysiloxane $(\equiv Si-O-)_n$) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ses atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques $CH_2$=CH-, ou encore la réaction entre des groupements thio-fonctionnels -SH et ces mêmes groupements vinyliques.

Des exemples de polymères à squelette polysiloxanique greffé par des monomères organiques non-siliconés convenant à une mise en oeuvre de la présente invention, ainsi que leur mode particulier de préparation, sont notamment décrits dans les demandes de brevets EP-A-0582152, WO 93/23009 et WO 95/03776.

Selon un mode particulièrement préféré de réalisation de la présente invention, le polymère siliconé, à squelette polysiloxanique greffé par des monomères organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélanges, parmi les esters d'acide acrylique d'alcanols et/ou les esters d'acide méthacrylique d'alcanols. Les alcanols sont de préférence en $C_1$-$C_{18}$ et plus particulièrement en $C_1$-$C_{12}$. Les monomères préférentiels sont choisis dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés à squelette polysiloxanique greffé par des monomères organiques non-siliconés convenant particulièrement bien à la mise en oeuvre de la présente invention est constituée par les polymères siliconés comportant dans leur structure le motif de formule (IV) suivant :

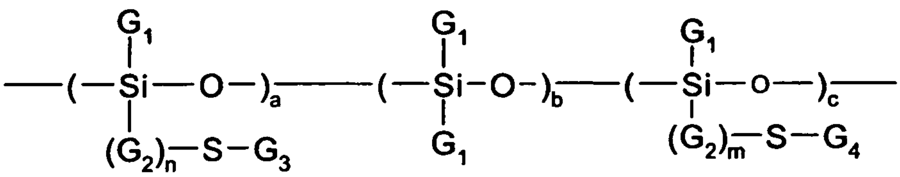

(IV)

dans lequel les radicaux $G_1$ , identiques ou différents, représentent l'hydrogène ou un radical alkyle en $C_1$-$C_{10}$ ou encore un radical phényle ; les radicaux $G_2$ , identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère hydrophobe à insaturation éthylénique ; m et n sont, indépendamment l'un de l'autre, égaux à 0 ou 1 ; a est un nombre entier allant de 0 et 50 ; b est un nombre entier compris entre 10 et 350, c est un nombre entier allant de 0 à 50 ; sous réserve que l'un des paramètres a et c soit différent de 0.

De préférence, le motif de formule (IV) ci-dessus présente au moins l'une, et encore plus préférentiellement l'ensemble, des caractéristiques suivantes :

- les radicaux $G_1$ désignent un radical alkyle en $C_1$-$C_{10}$ ;
- n est non nul, et les radicaux $G_2$ représentent un radical divalent en $C_1$-$C_3$ ;
- $G_3$ représente un radical polymérique résultant de l'(homo)polymérisation d'au moins un monomère du type acide carboxylique à insaturation éthylénique, de préférence l'acide acrylique et/ou l'acide méthacrylique ;
- $G_4$ représente un radical polymérique résultant de l'(homo)polymérisation d'au moins un monomère du type (méth) acrylate d'alkyle en $C_1$-$C_{10}$.

Des exemples de polymères siliconés greffés répondant à la formule (IV) sont ainsi notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et/ou du type poly(méth)acrylate d'alkyle.

Ces polymères sont référencés sous la dénomination CTFA "polysilicone-8".

Il peut ainsi s'agir d'un polydiméthyl siloxane greffé propylthio(polyacrylate de méthyle), propylthio(polyméthacrylate de méthyle) et propylthio(polyacide méthacrylique). En variante, il peut s'agir d'un polydiméthyl siloxane greffé propylthio(polyméthacrylate d'isobutyle) et propylthio(polyacide méthacrylique).

De tels polymères siliconés greffés sont notamment vendus par la Société 3M sous les dénominations commerciales VS 80, VS 70 ou LO21.

De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.

La composition utilisée selon l'invention peut se présenter sous toutes les formes galéniques normalement utilisées pour une application topique sur la peau, notamment sous forme d'une solution aqueuse, hydroalcoolique ou

huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

La composition utilisée selon l'invention constitue plus particulièrement une composition antirides, se présentant notamment sous forme d'un sérum.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants ainsi que leurs concentrations doivent être tels qu'ils ne modifient pas la propriété recherchée de tenseur du système polymérique.

Lorsque la composition utilisée selon l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut citer les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-50 et le stéarate de PEG-40, et les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols (glycérine, propylène glycol), les vitamines, les agents kératolytiques et/ou desquamants, les agents apaisants et leurs mélanges.

En outre, on peut aussi associer aux agents tenseurs utilisés selon l'invention d'autres composés connus de l'homme du métier comme agents tenseurs et ayant des propriétés différentes de celles des agents utilisés selon l'invention, notamment une protéine ou un hydrolysat de protéine. Comme composés de ce type, on peut citer par exemple les protéines de lait comme le lactalbumine, les protéines végétales telles que la protéine de soja vendue sous le nom d'Eleseryl par la société LSN, le dérivé d'avoine vendu sous la dénomination « Reductine » par la société Silab ou les acides nucléiques comme l'ADN. On peut en variante associer aux agents tenseurs selon l'invention les polymères tenseurs décrits dans FR-A-2 758 084.

Ainsi, la composition utilisée selon l'invention comprend de préférence, dans un milieu physiologiquement acceptable, au moins un polymère siliconé greffé tel que défini précédemment et une ou plusieurs protéines végétales.

En variante ou en plus, on préfère que la composition utilisée selon l'invention comprenne, dans un milieu physiologiquement acceptable, au moins un polymère siliconé greffé tel que défini précédemment et un ou plusieurs composés drainants, lipolytiques, désinfiltrants, amincissants, raffermissants, anti-glycants et/ou vaso-protecteurs.

Comme actifs amincissants, on peut utiliser dans la composition utilisée selon l'invention tous les composés connus en soi comme présentant une activité plus ou moins marquée dans le domaine de la lutte contre l'adiposité et/ou la cellulite et/ou comme amincissants du visage. Ils peuvent être choisis par exemple parmi :

1) les inhibiteurs de phosphodiestérase, tels que :

- les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline,

l'aminophylline, le chloroéthylthéophylline, le diprofylline, le diniprophylline, l'étamiphylline et ses dérivés, l'étofylline, la proxyphylline ;

- les associations contenant des dérivés xanthiques, comme l'association de caféine et de silanol (dérivé méthylsilanetriol de caféine), et par exemple le produit commercialisé par la société Exsymol sous la dénomination caféisilane C;

- les composés d'origine naturelle contenant des bases xanthiques et notamment de la caféine, tels que les extraits de thé, de café, de guarana, de maté, de cola (*Cola Nitida*) et notamment l'extrait sec de fruit de guarana (*Paulina sorbilis*) contenant 8 à 10 % de caféine ;

- l'éphédrine et ses dérivés qui peuvent notamment se retrouver à l'état naturel dans les plantes telles que le Ma Huang (Ephedra plant)

2) les extraits végétaux et les extraits d'origine marine, qui sont soit actifs sur les récepteurs à inhiber, tels que les $\alpha$2-bloqueurs, les NPY-bloqueurs, soit actifs pour stimuler les récepteurs $\beta$ et les protéines G, conduisant à l'activation de l'adénylcyclase, soit encore ceux agissant sur la lipoprotéine lipase, inhibant la captation du glucose ou agissant sur d'autres enzymes favorables à la lipolyse et/ou défavorables à la lipogénèse. Comme extraits végétaux de ce type, on peut citer par exemple :

- le *Garcinia Cambogia*,
- les extraits de *Bupleurum chinensis,*
- les extraits de lierre grimpant *(Hedera Helix),* d'arnica *(Arnica Montana L),* de romarin *(Rosmarinus officinalis N),* de souci *(Calendula officinalis),* de sauge *(Salvia officinalis L),* de ginseng *(Panax ginseng),* de millepertuis *(Byperycum Perforatum),* de fragon *(Ruscus aculeatus L),* d'ulmaire *(Filipendula ulmaria L),* d'orthosiphon *(Orthosiphon Stamincus Benth),* de bouleau *(Betula alba),*
- les extraits de ginkgo biloba,
- les extraits de prêle,
- les extraits d'escine,
- les extraits de cangzhu,
- les extraits de *chrysanthellum indicum,*
- les extraits des plantes du genre *Armeniacea, Atractylodis Platicodon, Sinommenum, Pharbitidis, Flemingia,*
- les extraits de *Coleus* tels que *C. Forskohlii, C. blumei, C. esquirolii, C. scutellaroïdes , C. xanthantus* et C. *Barbatus,* tel que l'extrait de racine de *Coleus Barbatus* contenant 60 % de forskoline,
- les extraits de Ballote,
- les extraits de *Guioa,* de *Davallia,* de *Terminalia,* de *Barringtonia,* de *Trema, d'Antirobia.*

Comme extrait d'origine marine on peut citer :

- les extraits d'algues, tels que l'extrait d'algue rouge (Gelidium cartilagineum) commercialisé par la société Secma sous la dénomination commerciale Rhodystérol, ou l'extrait de *Laminaria Digitata* commercialisé sous la dénomination PHYCOX75 par la société Secma,

- les protamines et leurs dérivés tels que ceux décrits dans le document FR-A-2,758,724.

3) Les actifs amincissants qui se lient aux PPARs (Peroxisome Proliferator activated receptor), tels que ceux décrits dans les documents WO-A-97/10813 et WO-96/33724, ou les actifs qui bloquent la différenciation des cellules précurseurs d'adipocytes dans le tissu adipeux.

La quantité d'actif(s) amincissant(s) peut varier dans une large mesure et dépend de la nature de ou des actifs utilisés. De manière générale, le ou les actifs amincissants sont présents en une concentration allant de 0,05 à 20 % et de préférence de 0,1 à 10 % en poids par rapport au poids total de la composition.

On peut ajouter à ces actifs amincissants des actifs qui vont compléter l'action des actifs amincissants, et notamment :

1) les actifs agissant sur la microcirculation (vasoprotecteur ou vasodilatateur), tels que les flavonoïdes, les ruscogénines, les esculosides, l'escine extraite du marron d'Inde, les nicotinates, l'hépéridine méthyl chalcone, le petit houx, les huiles essentielles de lavande ou de romarin, et certains dérivés de rutine tels que le sel disodique de

rutinyl sulfate ;

2) les actifs raffermissants et/ou antiglycant (empêchant la fixation du sucre sur les fibres de collagène), tels que les extraits de *centella asiatica* et de *siegesbeckia* et les extraits de levure du type *Saccharomyces cerevisiae,* qui stimulent la synthèse de collagène, ainsi que le silicium et l'amadorine, qui agissent comme anti-glycants.

Ainsi, la composition utilisée selon l'invention renferme avantageusement, dans un milieu physiologiquement acceptable, au moins un polymère siliconé greffé tel que défini précédemment et un ou plusieurs composés choisis parmi : un extrait de marron d'Inde, un extrait de lierre, un extrait de petit houx, un extrait de Bupleurum chinensis, un extrait d'algue, la caféine et un dérivé de rutine.

La quantité de ces actifs peut varier dans une large mesure. De manière générale, ces actifs sont présents en une concentration allant de 0,01 à 15 % et de préférence de 0,05 à 10 % en poids par rapport au poids total de la composition.

En cas d'incompatibilité, les actifs indiqués ci-dessus peuvent être incorporés dans des sphérules, notamment des vésicules ioniques ou non-ioniques et/ou des nanoparticules (nanocapsules et/ou nanosphères), de manière à les isoler les uns des autres dans la composition.

Les exemples ci-après de compositions utilisées selon l'invention sont donnés à titre d'illustration et sans caractère limitatif. Les quantités y sont données en pourcentages en poids.

**Exemple 1 : Crème antirides**

- Polyisobutène hydrogéné 5,5 %
- Néopentanoate d'isostéaryle 3,5 %
- Glycéryl stéarate et PEG-100 stéarate 2,0 %
- Alcools gras 3,5 %
- Acide stéarique 3,0 %
- Cyclométhicone 11,0 %
- Polyacrylamide et $C_{13}$-$C_{14}$ isoparaffine et laureth-7 (Sepigel 305 vendu par la société SEPPIC) 1,7 %
- Polysilicone-8 (VS 80 de 3M) en solution aqueuse à 30% 7,0 %
- Conservateurs 0,3 %
- Neutralisant 0,03%
- Eau déminéralisée qsp 100 %

**Exemple 2 : Sérum anti-âge**

- Polyacrylamide et $C_{13}$-$C_{14}$ isoparaffine et laureth-7 (Sepigel 305 vendu par la société SEPPIC) 1,0 %
- Gomme de xanthane 0,2 %
- Polymère d'anhydride maléique et de méthyl vinyl éther réticulé par le 1,9-décadiène 0,2 %
- Triéthanolamine 0,2 %
- Polysilicone-8 (VS 80 de 3M) en solution aqueuse à 30% 7,0 %
- Conservateurs 0,3 %
- Eau déminéralisée qsp 100 %

**Exemple 3 : Crème amincissante pour le visage**

| | |
|---|---|
| Polyisobutène hydrogéné | 5,0 % |
| Escine | 0,1 % |
| Caféine | 0,3 % |
| Glycérine et propylène glycol | 7 % |

Suite de tableau

| | |
|---|---|
| Cyclométhicone et diméthiconol | 2,2 % |
| Protéine de soja | 5 % |
| 0,1 % | 0,1 % |
| Gélifiant | 1 % |
| Polysilicone-8 (VS80 de 3M) en solution aqueuse à 30% | 1 % |
| Extrait de Gelidium cartilagineum (Rhodystérol de SECMA) | 2,5 % |
| Extrait de rhizome de Bupleurum chinensis et de caféine (Pleurimincyl de SEDERMA) | 2 % |
| Alcool | 15 % |
| Charges | 0,2 % |
| Neutralisant | 0,013 % |
| Conservateurs | 0,25 % |
| Eau | qsp. 100 % |

**Exemple 4 : Sérum galbeur pour le visage**

| | |
|---|---|
| Tétraoctanoate de pentaérythrityle | 5,0 % |
| Extrait de lierre grimpant | 0,1 % |
| Caféine | 0,5 % |
| Extrait de Terminalia sericea | 0,1 % |
| Glycérine | 3 % |
| Cyclométhicone | 2 % |
| Protéine de soja | 10 % |
| Sel disodique de rutinyl disulfate | 0,05 % |
| Gélifiants | 3 % |
| Polysilicone-8 (VS80 de 3M) en solution aqueuse à 30% | 1 % |
| Extrait de levure | 3,5 % |
| Alcool | 10 % |
| Neutralisant | 0,013 % |
| Conservateurs | 0,25 % |
| Eau | qsp. 100 % |

**Exemple 5 : Evaluation sensorielle de l'effet tenseur**

Le sérum de l'Exemple 2 ci-dessus a été testé sur un panel de cinq femmes présentant des rides et ridules au niveau du contour de l'oeil. Deux essais ont été effectués en appliquant le sérum sur le contour de l'oeil droit ou gauche, le contour de l'autre oeil étant laissé à nu. Par ailleurs, trois essais ont été effectués en appliquant une quantité de sérum différente sur les contours des deux yeux.

Il a été observé que l'effet tenseur variait, avec la quantité de sérum appliquée, d'un léger lissage des rides et ridules à un effet lissant/tenseur nettement plus visible. Les rides de la patte d'oie étaient ainsi réduites, ainsi que celles se trouvant sous l'oeil, notamment après séchage du film sur la peau, tout en offrant un toucher cosmétique doux, glissant et frais.

**Exemple 6 : Evaluation de l'effet tenseur par mesure au Dermomètre**

La composition et le sérum des exemples 1 et 2 ont été testés au Dermomètre. Cet appareil a été décrit par L. Rasseneur et al. dans Influence des Différents Constituants de la Couche Cornée sur la Mesure de son Elasticité, International Journal of Cosmetic Science, 4, 247-260 (1982).

Le principe de la méthode consiste à mesurer la longueur d'une éprouvette de stratum corneum isolé à partir d'une peau humaine provenant d'une opération chirurgicale, avant et après traitement avec les compositions à tester.

Pour ce faire, l'éprouvette est placée entre les deux mâchoires de l'appareil, dont l'une est fixe et l'autre mobile, dans une atmosphère à 30°C et 40% d'humidité relative.

On exerce une traction sur l'éprouvette, et on enregistre la courbe de la force (en grammes) en fonction de la longueur (en millimètres), la longueur zéro correspondant au contact entre les deux mors de l'appareil.

On trace ensuite la tangente à la courbe dans sa région linéaire. L'intersection de cette tangente avec l'axe des abcisses correspond à la longueur apparente $L_o$ de l'éprouvette à force nulle.

On détend l'éprouvette puis on applique sur le stratum corneum 2 mg/cm$^2$ de la composition à tester. Après 15 mn de séchage, les étapes ci-dessus sont à nouveau mises en oeuvre pour déterminer la longueur $L_1$ de l'éprouvette après traitement.

Le pourcentage de rétraction est défini par :

$$\% \text{ rétraction} = 100 \times (L_1 - L_o)/L_o$$

Pour caractériser un effet tenseur, ce pourcentage doit être négatif et l'effet tenseur est d'autant plus important que la valeur absolue du pourcentage de rétraction est élevée.

<u>Résultats :</u>

Le sérum selon l'Exemple 2 comprenant le polymère tenseur utilisée selon l'invention présente un pourcentage de rétraction de -1,8 ± 0,4% (moyenne et écart-type sur 7 échantillons), alors que le même sérum ne renfermant pas de polymère tenseur offre un pourcentage de rétraction -0,5 ± 0,3%. Ce résultat confirme l'effet tenseur du polymère utilisé dans ce sérum et dans la composition de l'Exemple 1.

## Revendications

1. Utilisation pour diminuer ou effacer les signes de vieillissement cutané, en particulier pour réduire ou effacer les rides et/ou les ridules de la peau et/ou pour lisser la peau, d'une composition comprenant au moins un polymère à squelette polysiloxanique greffé par au moins un monomère organique non-siliconé.

2. Utilisation selon la revendication 1, **caractérisée en ce que** ledit polymère comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente.

3. Utilisation selon la revendication 2, **caractérisée en ce que** ledit monomère anionique à insaturation éthylénique est choisi parmi les acides carboxyliques insaturés, linéaires ou ramifiés, éventuellement partiellement ou totalement neutralisés sous la forme d'un sel, et leurs mélanges.

4. Utilisation selon la revendication 3, **caractérisée en ce que** ledit acide carboxylique insaturé est choisi parmi l'acide acrylique, l'acide méthacrylique, l'acide maléique, l'anhydride maléique, l'acide itaconique, l'acide fumarique et l'acide crotonique.

5. Utilisation selon l'une quelconque des revendications 2 à 4, **caractérisée en ce que** ledit monomère hydrophobe à insaturation éthylénique est choisi parmi les esters d'acide acrylique d'alcanols et/ou les esters d'acide méthacrylique d'alcanols.

6. Utilisation selon la revendication 5, **caractérisée en ce que** ledit monomère hydrophobe à insaturation éthylénique est choisi dans le groupe constitué par le (méth)acrylate d'isooctyle, le (méth)acrylate d'isononyle, le 2-éthylhexyl (méth)acrylate, le (méth)acrylate de lauryle, le (méth)acrylate d'isopentyle, le (méth)acrylate de n-butyle, le (méth) acrylate d'isobutyle, le (méth)acrylate de méthyle, le (méth)acrylate de tertio-butyle, le (méth)acrylate de tridécyle, le (méth)acrylate de stéaryle et leurs mélanges.

7. Utilisation selon l'une quelconque des revendications 1 à 6, **caractérisée en ce que** ledit polymère siliconé comporte dans sa structure le motif de formule (IV) suivant :

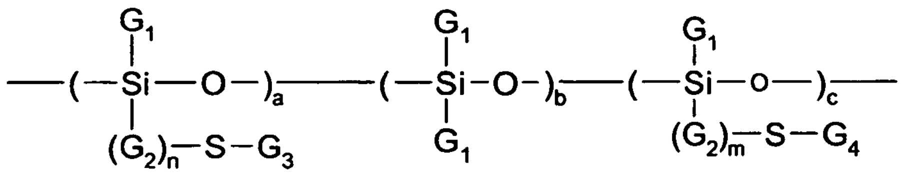

(IV)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère hydrophobe à insaturation éthylénique ; m et n sont, indépendamment l'un de l'autre, égaux à 0 ou 1 ; a est un nombre entier allant de 0 et 50 ; b est un nombre entier compris entre 10 et 350, c est un nombre entier allant de 0 et 50 ; sous réserve que l'un des paramètres a et c soit différent de 0.

**8.** Utilisation selon la revendication 7, **caractérisée en ce que** ledit motif de formule (IV) présente au moins l'une, et de préférence l'ensemble, des caractéristiques suivantes :

- les radicaux $G_1$ désignent un radical alkyle en $C_1$-$C_{10}$ ;
- n est non nul, et les radicaux $G_2$ représentent un radical divalent en $C_1$-$C_3$ ;
- $G_3$ représente un radical polymérique résultant de l'(homo)polymérisation d'au moins un monomère du type acide carboxylique à insaturation éthylénique ;
- $G_4$ représente un radical polymérique résultant de l'(homo)polymérisation d'au moins un monomère du type (méth)acrylate d'alkyle en $C_1$-$C_{10}$.

**9.** Utilisation selon la revendication 8, **caractérisée en ce que** ledit polymère siliconé greffé répondant à la formule (IV) est un polydiméthylsiloxane sur lequel sont greffés, par l'intermédiaire d'un chaînon de raccordement de type thiopropylène, des motifs polymères mixtes du type acide poly(méth)acrylique et du type poly(méth)acrylate d'alkyle.

**10.** Utilisation selon l'une quelconque des revendications précédentes, **caractérisée en ce que** ledit polymère siliconé greffé représente de 0,03 à 25% , de préférence de 0,3 à 6%, mieux, environ 2%, du poids total de ladite composition.

## Claims

**1.** Use in decreasing or removing signs of cutaneous ageing, in particular in reducing or removing wrinkles and/or fine lines of the skin and/or in smoothing out the skin, of a composition comprising at least one polymer with a polysiloxane backbone grafted by at least one non-silicone organic monomer.

**2.** Use according to Claim 1, **characterized in that** the said polymer comprises the result of the radical copolymerization between, on the one hand, at least one anionic non-silicone organic monomer exhibiting an ethylenic unsaturation and/or one hydrophobic non-silicone organic monomer exhibiting an ethylenic unsaturation and, on the other hand, a silicone exhibiting, in its chain, at least one functional group capable of reacting with the said ethylenic unsaturations of the said non-silicone monomers with the formation of a covalent bond.

**3.** Use according to Claim 2, **characterized in that** the said anionic monomer with ethylenic unsaturation is chosen from linear or branched unsaturated carboxylic acids, optionally partially or completely neutralized in the form of a salt, and their mixtures.

**4.** Use according to Claim 3, **characterized in that** the said unsaturated carboxylic acid is chosen from acrylic acid, methacrylic acid, maleic acid, maleic anhydride, itaconic acid, fumaric acid and crotonic acid.

**5.** Use according to any one of Claims 2 to 4,
**characterized in that** the said hydrophobic monomer with ethylenic unsaturation is chosen from alkanol acrylic acid esters and/or alkanol methacrylic acid esters.

6. Use according to Claim 5, **characterized in that** the said hydrophobic monomer with ethylenic unsaturation is chosen from the group composed of isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate, stearyl (meth)acrylate and their mixtures.

7. Use according to any one of Claims 1 to 6,
**characterized in that** the said silicone polymer comprises, in its structure, the unit of following formula (IV):

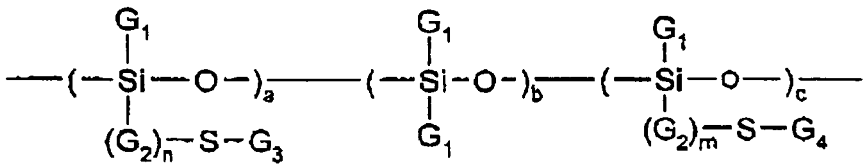

(IV)

in which the $G_1$ radicals, which are identical or different, represent hydrogen or a $C_1$-$C_{10}$ alkyl radical or alternatively a phenyl radical; the $G_2$ radicals, which are identical or different, represent a $C_1$-$C_{10}$ alkylene group; $G_3$ represents a polymer residue resulting from the (homo)polymerization of at least one anionic monomer with ethylenic unsaturation; $G_4$ represents a polymer residue resulting from the (homo)polymerization of at least one hydrophobic monomer with ethylenic unsaturation; m and n are, independently of one another, equal to 0 or 1; a is an integer ranging from 0 to 50; b is an integer between 10 and 350 and c is an integer ranging from 0 to 50, with the proviso that one of the parameters a and c is other than 0.

8. Use according to Claim 7, **characterized in that** the said unit of formula (IV) exhibits at least one, and preferably all, of the following characteristics:

- the $G_1$ radicals denote a $C_1$-$C_{10}$ alkyl radical;
- n is not zero and the $G_2$ radicals represent a divalent $C_1$-$C_3$ radical;
- $G_3$ represents a polymer radical resulting from the (homo)polymerization of at least one monomer of the carboxylic acid with ethylenic unsaturation type;
- $G_4$ represents a polymer radical resulting from the (homo)polymerization of at least one monomer of the ($C_1$-$C_{10}$) alkyl (meth)acrylate type.

9. Use according to Claim 8, **characterized in that** the said grafted silicone polymer corresponding to the formula (IV) is a polydimethylsiloxane to which are grafted, via a connecting link of thiopropylene type, mixed polymer units of the poly((meth)acrylic acid) type and of the poly(alkyl (meth)acrylate) type.

10. Use according to any one of the preceding claims, **characterized in that** the said grafted silicone polymer represents from 0.03 to 25%, preferably from 0.3 to 6%, better still approximately 2%, of the total weight of the said composition.

**Patentansprüche**

1. Verwendung einer Zusammensetzung, die mindestens ein Polymer mit Polysiloxan - Grundgerüst enthält, auf das mindestens ein organisches, nicht siliconhaltiges Monomer gepfropft ist, um die Zeichen der Hautalterung zu vermindern oder verschwinden zu lassen, und insbesondere um Falten und/oder Fältchen der Haut zu reduzieren oder verschwinden zu lassen und/oder um die Haut zu glätten.

2. Verwendung nach Anspruch 1, **dadurch gekennzeichnet, dass** das Polymer bei der radikalischen Copolymerisation einerseits mindestens eines organischen, anionischen und nicht siliconhalten Monomers, das eine ethylenisch ungesättigte Bindung enthält, und/oder eines organischen, hydrophoben und nicht siliconhaltigen Monomers, das eine ethylenisch ungesättigte Bindung enthält, und andererseits eines Silicons, das in seiner Kette mindestens eine funktionelle Gruppe aufweist, die in der Lage ist, mit den ethylenisch ungesättigten Bindungen der oben genannten nicht siliconhaltigen Monomere eine kovalente Bindung einzugehen, gebildet wird.

3. Verwendung nach Anspruch 2, **dadurch gekennzeichnet, dass** das anionische Monomer mit der ethylenisch ungesättigten Bindung unter den linearen oder verzweigten, ungesättigten Carbonsäuren und ihren Gemischen

ausgewählt ist, die gegebenenfalls teilweise oder ganz in Form eines Salzes neutralisiert sind.

4. Verwendung nach Anspruch 3, **dadurch gekennzeichnet, dass** die ungesättigte Carbonsäure unter Acrylsäure, Methacrylsäure, Maleinsäure, Maleinsäureanhydrid, Itaconsäure, Fumarsäure und Crotonsäure ausgewählt ist.

5. Verwendung nach einem der vorhergehenden Ansprüche 2 bis 4, **dadurch gekennzeichnet, dass** das hydrophobe Monomer mit der ethylenisch ungesättigten Bindung unter den Alkanolacrylsäureestern und/oder den Alkanolmethacrylsäureestern ausgewählt ist.

6. Verwendung nach Anspruch 5, **dadurch gekennzeichnet, dass** das hydrophobe Monomer mit der ethylenisch ungesättigten Bindung unter Isooctyl(meth)acylat, Isononyl(meth)acrylat, 2-Ethylhexyl(meth)acrylat, Lauryl(meth) acrylat, Isopentyl(meth)acrylat, *n*-Butyl(meth)acrylat, Isobutyl(meth)acrylat, Methyl(meth)acrylat, *t*-Butyl(meth)acrylat, Tridecyl(meth)acrylat, Stearyl(meth)acrylat und ihren Gemischen ausgewählt ist.

7. Verwendung nach einem der Ansprüche 1 bis 6, **dadurch gekennzeichnet, dass** das Siliconpolymer in seiner Struktur die Einheit der folgenden Formel (IV) aufweist:

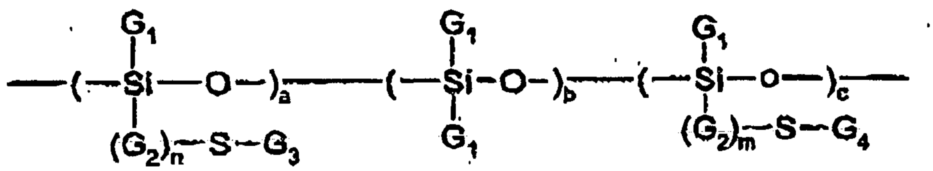

(IV),

in der die Reste $G_1$, die gleich oder verschieden sind, ein Wasserstoffatom, eine $C_{1-10}$-Alkylgruppe oder eine Phenylgruppe bedeuten;
die Reste $G_2$, die gleich oder verschieden sind, eine $C_{1-10}$-Alkylengruppe darstellen;
$G_3$ eine Polymergruppe bedeutet, die aus der (Homo)polymerisation mindestens eines anionischen Monomers mit einer ethylenisch ungesättigten Bindung entstanden ist;
$G_4$ einen Polymergruppe bedeutet, die aus der (Homo)polymerisation mindestens eines hydrophoben Polymers mit einer ethylenisch ungesättigten Bindung entstanden ist;
m und n jeweils unabhängig voneinander 0 oder 1 sind;
a Null oder eine ganze Zahl im Bereich von 1 und 50 ist;
b eine ganze Zahl im Bereich von 10 und 350 ist, c Null oder eine ganze Zahl im Bereich von 1 und 50 ist, unter der Voraussetzung, dass einer der Parameter a und c ungleich 0 ist.

8. Verwendung nach Anspruch 7, **dadurch gekennzeichnet, dass** die Einheit (IV) mindestens eines und vorzugsweise alle der folgenden Merkmale aufweist:

   - die Reste $G_1$ bedeuten eine $C_{1-10}$-Alkylgruppe;
   - n ist nicht Null und die Reste $G_2$ bedeuten eine zweiwertige $C_{1-3}$-Gruppe;
   - $G_3$ bedeutet eine Polymergruppe, die aus der (Homo)-polymerisation mindestens eines Monomers vom Carbonsäuretyp mit einer ethylenisch ungesättigten Bindung gebildet wird;
   - $G_4$ bedeutet eine Polymergruppe, die aus der (Homo)-polymerisation mindestens eines Monomers vom $C_{1-10}$-Alkyl(meth)acrylattyp entstanden ist.

9. Verwendung nach Anspruch 8, **dadurch gekennzeichnet, dass** das Siliconpfropfpolyrner der Formel (IV) ein Polydimethylsiloxan ist, auf das über eine Verbindungsgruppe vom Thiopropylentyp gemischte Polymereinheiten vom Poly(meth)acrylsäure- und Alkylpoly(meth)acrylat - Typ gepfropft sind.

10. Verwendung nach einem der vorhergehenden Ansprüche, **dadurch gekennzeichnet, dass** das Siliconpfropfpolymer 0,03 bis 25 Gew.-%, vorzugsweise 0,3 bis 6 Gew.-%, besser etwa 2 Gew.-%, bezogen auf das Gesamtgewicht der Zusammensetzung, ausmacht.